# EUROPEAN PATENT SPECIFICATION

(11) **EP 1 179 046 B1**
(45) Date of publication and mention of the grant of the patent: **08.03.2006**
(21) Application number: 00930442.9
(22) Date of filing: 05.05.2000
(51) Int. Cl.: C12N 1/00, C12N 5/06, C12N 5/10

(54) **LINEAGE-RESTRICTED PRECURSOR CELLS ISOLATED FROM MOUSE NEURAL TUBE AND MOUSE EMBRYONIC STEM CELLS**
AUF EINE ZELLINIE BESCHRÄNKTE VORLÄUFERZELLEN AUS DEM NEURALROHR DER MAUS UND AUS EMBRYONALEN STAMMZELLEN DER MAUS
CELLULES PRECURSEURS A RESTRICTION DE LIGNAGE, ISOLEES A PARTIR DU TUBE NEURAL DE SOURIS ET DE CELLULES SOUCHES EMBRYONNAIRES DE SOURIS

(30) Priority: 07.05.1999 US 133159 P
(43) Date of publication of application: 13.02.2002
(73) Proprietor: University of Utah Research Foundation, Salt Lake City, Utah 84112 (US)
(72) Inventor: MUJTABA, Tahmina, Sandy, UT 84070 (US); RAO, Mahendra, S., Salt Lake City, UT 84124 (US)
(74) Representative: Suèr, Steven Johannes
(86) International application number: PCT/US2000/012446
(87) International publication number: WO 2000/068359

(56) References cited:
- WO-A-99/01159
- US-A- 5 411 883
- US-A- 5 688 692
- US-A- 5 753 506
- MUJTABA T ET AL: "Lineage-restricted neural precursors can be isolated from both the mouse neural tube and cultured ES cells." DEVELOPMENTAL BIOLOGY, vol. 214, no. 1, 1 October 1999 (1999-10-01), pages 113-127, XP002216609 ISSN: 0012-1606
- BAILEY K A ET AL: "NEURONAL PROGENITORS IDENTIFIED BY THEIR INABILITY TO EXPRESS CLASSI HISTOCOMPATIBILITY ANTIGENS IN RESPONSE TO INTERFERON-GAMMA" JOURNAL OF NEUROSCIENCE RESEARCH, WILEY-LISS, US, vol. 39, no. 2, October 1994 (1994-10), pages 166-167, XP002917554 ISSN: 0360-4012
- DATABASE BIOSIS [Online] BIOSCIENCES INFORMATION SERVICE, PHILADELPHIA, PA, US; April 1998 (1998-04) KIYOSHI ET AL.: "Gap junctional intercellular communication during neuronal differentiation of mouse embryonic stem cells under dispersed culture in vitro." Database accession no. PREV199800484151 XP002228953
- KILPATRICK T J ET AL: "CLONED MULTIPOTENTIAL PRECURSOR FROM THE MOUSE CEREBRUM REQUIRE FGF-2, WHEREAS GLIAL RESTRICTED PRECURSORS ARE STIMULATED WITH EITHER FGF-2 OR EGF" JOURNAL OF NEUROSCIENCE, NEW YORK, NY, US, vol. 15, no. 5, 1995, pages 3653-3661, XP000916277 ISSN: 0270-6474
- RAO MAHENDRA S ET AL: "A tripotential glial precursor cell is present in the developing spinal cord." PROCEEDINGS OF THE NATIONAL ACADEMY OF SCIENCES OF THE UNITED STATES, vol. 95, no. 7, 31 March 1998 (1998-03-31), pages 3996-4001, XP002228951 March 31, 1998 ISSN: 0027-8424
- O'SHEA H ET AL: "The P1 capsid region of Theiler's virus controls replication in mouse glial cell cultures." ARCHIVES OF VIROLOGY, vol. 142, no. 8, 1997, pages 1521-1535, XP002228952 ISSN: 0304-8608

### SEQUENCE LISTING

<110> Mujtaba, Tahmina
   Rao, Mahendra S.
   University of Utah Research Foundation
<120> Lineage-Restricted Precursor Cells Isolated from Mouse Neural Tube and Mouse Embryonic Stem Cells
<130> UT-0025
<140>
   <141>
<150> 60/133,159
   <151> 1999-05-07
<160> 28
<170> Patent In Ver. 2.1
<210> 1
   <211> 21
   <212> DNA
   <213> Artificial Sequence
<220>
   <223> Description of Artificial Sequence: Synthetic
<400> 1
   gcacatactc agacgaagcc a 21
<210> 2
   <211> 24
   <212> DNA
   <213> Artificial Sequence
<220>
   <223> Description of Artificial Sequence: Synthetic
<400> 2
   agcagccaag atggagcaat agac 24
<210> 3
   <211> 23
   <212> DNA
   <213> Artificial Sequence
<220>
   <223> Description of Artificial Sequence: Synthetic
<400> 3
   ctgaatactg gctgaatgac atg 23
<210> 4
   <211> 23
   <212> DNA
   <213> Artificial Sequence
<220>
   <223> Description of Artificial Sequence: Synthetic
<400> 4
   aaattaatga caacatccaa gac 23
<210> 5
   <211> 20
   <212> DNA
   <213> Artificial Sequence
<220>
   <223> Description of Artificial Sequence: Synthetic
<400> 5
   gcagcatagg cttcagcaag 20
<210> 6
   <211> 19
   <212> DNA
   <213> Artificial Sequence
<220>
   <223> Description of Artificial Sequence: Synthetic
<400> 6
   gtagcaggtc cgcaaggtg 19
<210> 7
   <211> 21
   <212> DNA
   <213> Artificial Sequence
<220>
   <223> Description of Artificial Sequence: Synthetic
<400> 7
   gaatcttttc tcctggtggt g                                              21
<210> 8
   <211> 21
   <212> DNA
   <213> Artificial Sequence
<220>
   <223> Description of Artificial Sequence: Synthetic
<400> 8
   gatcaaaagc cccgtacaca g                                              21
<210> 9
   <211> 18
   <212> DNA
   <213> Artificial Sequence
<220>
   <223> Description of Artificial Sequence: Synthetic
<400> 9
   gcagaatccc acctgcag                                                  18
<210> 10
   <211> 19
   <212> DNA
   <213> Artificial Sequence
<220>
   <223> Description of Artificial Sequence: Synthetic
<400> 10
   gttgctggca tcgaaagag                                                 19
<210> 11
   <211> 24
   <212> DNA
   <213> Artificial Sequence
<220>
   <223> Description of Artificial Sequence: Synthetic
<400> 11
   gcacagacat ggttgggata ctag 24
<210> 12
   <211> 20
   <212> DNA
   <213> Artificial Sequence
<220>
   <223> Description of Artificial Sequence: Synthetic
<400> 12
   gcagggctgt tctggagtcg 20
<210> 13
   <211> 20
   <212> DNA
   <213> Artificial Sequence
<220>
   <223> Description of Artificial Sequence: Synthetic
<400> 13
   ccaccgtgtt cttcgacatc 20
<210> 14
   <211> 19
   <212> DNA
   <213> Artificial Sequence
<220>
   <223> Description of Artificial Sequence: Synthetic
<400> 14
   ggtccagcat ttgccatgg 19
<210> 15
   <211> 21
   <212> DNA
   <213> Artificial Sequence
<220>
   <223> Description of Artificial Sequence: Synthetic
<400> 15
   gctggggaag aggagaggag a 21
<210> 16
   <211> 22
   <212> DNA
   <213> Artificial Sequence
<220>
   <223> Description of Artificial Sequence: Synthetic
<400> 16
   acgagtggtg ggcaggtgtc tt 22
<210> 17
   <211> 23
   <212> DNA
   <213> Artificial Sequence
<220>
   <223> Description of Artificial Sequence: Synthetic
<400> 17
   tgggagcatc aaccacacct acc 23
<210> 18
   <211> 19
   <212> DNA
   <213> Artificial Sequence
<220>
   <223> Description of Artificial Sequence: Synthetic
<400> 18
   gcccgaagca gccctcgcc 19
<210> 19
   <211> 20
   <212> DNA
   <213> Artificial Sequence
<220>
   <223> Description of Artificial Sequence: Synthetic
<400> 19
   atcggaggca ttcggctgcg 20
<210> 20
   <211> 20
   <212> DNA
   <213> Artificial Sequence
<220>
   <223> Description of Artificial Sequence: Synthetic
<400> 20
   agaactgccg ggccaaaggg 20
<210> 21
   <211> 22
   <212> DNA
   <213> Artificial Sequence
<220>
   <223> Description of Artificial Sequence: Synthetic
<400> 21
   gacatgaagc tctcactggc ac 22
<210> 22
   <211> 21
   <212> DNA
   <213> Artificial Sequence
<220>
   <223> Description of Artificial Sequence: Synthetic
<400> 22
   catacattct ggcatcagcg c 21
<210> 23
   <211> 20
   <212> DNA
   <213> Artificial Sequence
<220>
   <223> Description of Artificial Sequence: Synthetic
<400> 23
   gaaggaaagg caccacactg 20
<210> 24
   <211> 18
   <212> DNA
   <213> Artificial Sequence
<220>
   <223> Description of Artificial Sequence: Synthetic
<400> 24
   gctggcgatg gtggtggg 18
<210> 25
   <211> 24
   <212> DNA
   <213> Artificial Sequence
<220>
   <223> Description of Artificial Sequence: Synthetic
<400> 25
   gccgagcaga ccaaggaggc catt 24
<210> 26
   <211> 24
   <212> DNA
   <213> Artificial Sequence
<220>
   <223> Description of Artificial Sequence: Synthetic
<400> 26
   ctggatggtg tcctggtagc tgct 24
<210> 27
   <211> 24
   <212> DNA
   <213> Artificial Sequence
<220>
   <223> Description of Artificial Sequence: Synthetic
<400> 27
   ttgcagacct cacagacgct gcgt 24
<210> 28
   <211> 24
   <212> DNA
   <213> Artificial Sequence
<220>
   <223> Description of Artificial Sequence: Synthetic
<400> 28
   cggttttctt cgccctccag caat 24

## Description

### Field of the Invention

The present invention relates to multipotent neuroepithelial (NEP) stem cells, and lineage-restricted, self-renewing precursor cells termed neuron-restricted precursors (NRPs) and glial-restricted precursors (GRPs) isolated from mouse neural tubes. Immunoselection against the cell surface markers E-NCAM and A2B5 were used to isolate mouse NRPs and GRPs, respectively. The present invention also relates to lineage-restricted precursor cells isolated from mouse embryonic stem cells (ES cells). As demonstrated herein, mouse ES cell-derived NRPs are E-NCAM immunoreactive, undergo self renewal in defined medium and differentiate into multiple neuronal phenotypes in mass culture. Mouse ES cells also generate A2B5 immunoreactive cells that are similar to GRPs derived from mouse neural tubes and which differentiate into oligodendrocytes and astrocytes. The present invention also relates to methods for generating these lineage-restricted precursors in vitro from mouse neural tubes and cultured mouse ES cells.

### Background of the Invention

Pluripotent stem cells in the central nervous system (CNS) generate differentiated neurons and glia either directly or through the generation of lineage-restricted intermediate precursors (see e.g. Kilpatrick, T. J. and Bartlett, B. F. J. Neurosci. 1995 15:3653-3661; Kilpatrick, T. J. and Bartlett, B. F. Neuron 1993 10:255-265; Price et al. Brain Pathol. 1992 2:23-29; Reynolds et al. J. Neurosci. 1992 12:4565-4574; Reynolds, B. A. and Weiss, S. Dev. Biol. 1996 175:1-13; Temple, S. and Davis, A. Development 1994 120:999-1008). Review of the relevant art indicates that different stem cells and lineage-restricted precursors with distinguishing characteristics and abilities are obtained depending upon the species, the region of the central nervous system from which the cells were obtained, and age of the animal from which the cells were obtained.

For example, multipotent neuroepithelial (NEP) cells have been shown to be present in the spinal cord of rats at embryonic day 10.5. These rat NEP cells are self-renewing cells that can differentiate into neurons, oligodendrocytes, astrocytes (Kalyani et al. Dev. Biol. 1997 186:202-223), and peripheral nervous system (PNS) derivatives (Mujtaba et al. Dev. Biol. 1998 200:1-15). Differentiation of rat NEP cells occurs via the generation of more restricted precursors. At embryonic day 13.5, two major types of lineage-restricted precursors are present in the rat: an E-NCAM immunoreactive neuronal precursor, termed NRP, that can generate multiple kinds of neurons but not oligodendrocytes or astrocytes (Mayer-Proschel et al. Neuron 1997 19:773-785; Kalyani et al. J. Neurosci. 1998 18:7856-7868); and an A2B5 immunoreactive glial precursor termed GRP, that can generate oligodendrocytes and astrocytes, but cannot generate neurons (Rao, M. S. and Mayer-Proschel, M. Dev. Biol. 1997 188:48-63; Mayer-Proschel et al. Neuron 1997 19:773-785; Rao et al. Proc. Natl. Acad. Sci. USA 1998 95:3996-4001).

U.S. Patent 5,753,506 by Johe et al. describes a different multipotential stem cell isolated from adult rat brain and regions of the embryonic rat brain including the cortex and striatum at embryonic day 14 and the hippocampus at embryonic day 16, as well as human fetal brain. This stem cell also undergoes self-renewal and can differentiate into neurons, astrocytes and oligodendrocytes upon culture in appropriate differentiation conditions. Cells isolated from rat embryonic cortex and striatum at embryonic day 14 and rat embryonic hippocampus at embryonic day 16 were 70% pure. These cells can be maintained in the presence of FGF as well as EGF and/or TGF alpha. PDGF acts on these stem cells to bias neuronal differentiation.

Cattaneo and McKay (Nature 1990 347: 762) disclose a rat neuron-restricted precursor cell isolated from early embryonic brain, more specifically rat embryonic striatum primordia from embryonic day 13.5-14.5. A greater than 95% population of rat striatal cells expressing nestin is disclosed.

Gard et al. (Developmental Biology 1995 167:596) disclose a glial-restricted precursor cell isolated from the optic nerve of 4 day old rats which are bipotential, generating oligodendrocytes and A2B5⁺/GFAP⁺ astrocytes.

U.S. Patent 5,411,883 by Boss et al. teaches cells isolated from ventral mesencephalon tissue from porcine or human embryos at Carnegie stages 15-18. The porcine precursor cells are demonstrated to differentiate into dopaminergic neurons.

The existence of spinal cord NRPs in chick embryos has been demonstrated by retroviral tracing (Leber et al. J. Neurosci. 1990 10:1688-1697; Leber, S.M. and Sanes, J.R. J. Neurosci. 1995 15:1236-1248).

In addition, NRP cell lines that are E-NCAM immunoreactive have been generated from human spinal cord cultures.

Multipotent stem cells have also been identified in the mouse cortex in embryos between 12 and 18 days of gestation (Williams, B.P. and Price, J. Neuron 1995 14:1181-88); the telencephalon and mesencephalon of mice at embryonic day 10 and the cerebrum of mice at embryonic day 17 (Kilpatrick, T. J. and Bartlett, B. F. J. Neurosci. 1995 15:3653-3661); adult mouse thoracic spinal cord (Weiss et al. J. Neurosci. 1996 16:7599-7609); and adult and embryonic forebrain of the mouse (Weiss et al. Trends Neurosci. 1996 19:387-393).

An O-2A-like glial progenitor cell has also been identified in the optic nerve and cortex at late embryonic stages (reviewed in Collarini et al. J. Cell Sci. Suppl. 1991 15:117-123). U.S. Patent 5,688,692 by Jat discloses a method for isolating a population of glial precursors from cortical cells derived from embryonic day 18 mice.

Neuronally restricted precursor cells from the juvenile subventricular zone (svz) of mice have also been identified by clonal analysis *in vivo* and *in vitro* (Young, G.M. and Levison, S.W. Dev. Neurosci. 1996 18:255-265).

However, all of these cells differ significantly in growth factor requirements, culture conditions, and differentiation potential from rat NEP cells identified by Kalyani et al. (Dev. Biol. 1997 186:202-223) and rat GRP and rat NRP cells identified by Mayer-Proschel et al. (Neuron 1997 19:773-785) and Kalyani et al. (J. Neurosci. 1998 18:7856-7868) and Rao, M. S. and Mayer-Proschel, M. (Dev. Biol. 1997 188:48-63), Mayer-Proschel et al. (Neuron 1997 19:773-785) and Rao et al. (Proc. Natl. Acad. Sci. USA 1998 95:3996-4001), respectively.

### Summary of the Invention

An object of the present invention is to provide an isolated, pure population of mouse glial-restricted precursor cells derived from mouse neural tubes at embryonic day 12.0 or mouse embryonic stem cells by A2B5 immunoreactivity. The mouse glial-restricted precursor cells self-renew. Under differentiating conditions, these mouse GRP cells differentiate into oligodendrocytes and at least two distinct populations of astrocytes, but not into neurons.

Another object of the present invention is to provide methods for isolating pure populations of the mouse glial-restricted precursor cells. In one embodiment, a pure population of mouse glial-restricted precursor cells is isolated by immunoselecting A2B5-immunoreactive cells from mouse E12.0 neural tube cells. In another embodiment, a pure population of mouse glial-restricted precursor cells is isolated by incubating mouse embryonic stem cells under differentiation-inducing conditions so that the cells differentiate and immunoselecting A2B5-immunoreactive cells from the differentiating cells.

### Detailed Description of the Invention

The present invention relates to purified neuroepithelial (NEP) cells, neuroblasts and glioblasts isolated from the developing mouse spinal cord and methods for isolating pure populations of these cells from the developing mouse spinal cord. The present invention also relates to pure populations of neuron- and glial-restricted precursor cells isolated from mouse embryonic stem (ES) cell cultures, as well as methods for isolating these cells from mouse ES cells. As demonstrated herein these cells exhibit characteristics similar to those described for rat neuronal precursor cells and different from previously described mouse neurosphere cells. In the present invention, E-NCAM immunoreactivity is used to isolate pure populations of differentiated mouse ES cells which generate multiple neuronal phenotypes, but which do not generate astrocytes or oligodendrocytes. A2B5 immunoreactivity is used to isolate pure populations of differentiated mouse ES which generate oligodendrocytes and astrocytes, but which do not generate neurons.

Mouse NEP cells are different from classes of mouse stem cells described previously. The properties of mouse NEP cells resemble those of rat NEP cells rather than other classes of stem cells isolated from other portions of the CNS and at different stages in development such as striatal embryonic cells described by Reynolds et al. (J. Neurosci 1992 12:4565-4574), multipotential precursors from the mouse cerebrum described by Kilpatrick, T.J. and Bartlett, B.F. (J. Neurosci. 1995 15:3653-3661), and multipotential stem cells from the adult mouse brain described by Gritti et al. (J. Neurosci 1996 16:1091-1100).

For example, as demonstrated herein, mouse NEP cells grow in adherent culture, do not express EGF-R immunoreactivity, survive and/or proliferate in EGF and absolutely require FGF for both proliferation and survival. Further, PDGF, which has been shown to be an instructive molecule for stem cells derived from the cortical region in mice (see e.g. Williams et al. Neuron 1997 18:553-562 describing the role of PDGF in ventricular zone progenitor cells), has no observable effect on the mouse NEP cells of the present invention, either alone or in combination with FGF.

In these experiments, the antigenic properties, growth characteristics and differentiation potential of these early mouse NEP precursor cells were examined. Specifically, mouse neural tubes at embryonic day 8.5 (E8.5) were isolated and cells were plated at low density to test the growth characteristics of NEP stem cells. NEP cells appeared homogeneous and were nestin immunoreactive. Virtually all cells incorporated BrdU over a 6 hour period indicating that they were actively dividing cells when maintained in 25 ng/ml of FGF. NEP cells did not express any markers of early differentiation as tested by either immunocytochemistry or RT-PCR. In particular, NEP cells did not express E-NCAM, β-111 tubulin or A2B5 immunoreactivity, thus indicating that they were undifferentiated precursor cells. Nestin immunoreactive cells persisted around the ventricular zone at E 12.0, while β-111 tubulin immunoreactive neurons appeared to differentiate at the ventricular margins.

Growth factor dependence of E8.5 NEP cells was tested in several growth factors at a variety of different concentrations. FGF was sufficient to maintain NEP cell proliferation. In contrast, NGF, PDGF and EGF were ineffective at any dose tested. Further, none of the growth factors tested acted synergistically with FGF to alter cell division rates thus suggesting that EGF and PDGF are neither survival or proliferation factors for the mouse NEP cells of the present invention. The failure to see a response to EGF is indicative of these cells being a distinct population from both EGF dependent neurospheres and FGF/EGF dependent cells isolated from other regions and later stages in the mouse (Reynolds et al. J. Neurosci. 1992 12:4565-4574; Reynolds, B.A. and Weiss, S. Dev. Biol. 1996 175:1-13; and Weiss et al. J. Neurosci. 1996 16:7599-7609).

Differences of the E8.5 mouse NEP cells were confirmed by examination of EGF-R expression in cell culture. EGF-R is not detected in the mouse NEP cells of the present invention. In contrast, EGF-R was readily detected in whole brain, as well as on glial precursors, neurons and astrocytes. Identical results were obtained using an antibody specific to mouse EGF-R. Accordingly, the cells of the present invention are clearly distinct from EGF dependent mouse neurospheres as well as FGF/EGF dependent mouse stem cells isolated at later stages of development.

The E8.5 mouse NEP cells can also be maintained in defined medium with the addition of FGF for multiple passages (at least ten) and still be induced to differentiate by replating the cells on laminin or poly-L-lysine and reducing FGF concentrations. NEP cells under these conditions readily differentiated into neurons, astrocytes and oligodendrocytes. Thus, the mouse NEP cells are FGF dependent, grow in adherent culture and can generate CNS derivatives. In addition, these cells generate p75 immunoreactive crest cells, SMA immunoreactive smooth muscle cells and peripheral glial and neurons. PCR analysis also confirmed an increase in the expression of p75 when NEP cells were induced to differentiate into neural crest by the addition of 10 ng/ml of BMP-4. Neural crest and its derivatives have not been generated from EGF dependent stem cells.

Thus, results from these experiments indicate that at least during early time periods of development, only FGF dependent pluripotent stem cells are present in the developing mouse spinal cord. Taken together with evidence that at later stages an EGF or EGF/FGF dependent cell is present, it is believed that at least two different classes of stem cells exist. Since the NEP cells of the present invention do not express EGF-R immunoreactivity, it is believed that EGF-R can serve as a marker for selection between these two different populations of stem cells.

As also demonstrated herein, E-NCAM and A2B5 immunoreactivity can be used to isolate lineage-restricted precursor cells from the mouse neural tube.

Differentiation in rat neural tubes has been shown to involve progressive restriction of cell fate (Mayer-Proschel et al. Neuron 1997 19:773-785; Kalyani et al. J. Neurosci. 1998 18:7856-7868). Rat NEP cells generate more restricted multipotent precursors both *in vitro* and *in vivo* (Mayer-Proschel et al. Neuron 1997 19:773-785; Rao et al. Proc. Natl Acad. Sci. USA 1998 95:3996-4001). Two such restricted multipotent precursors have been described: an E-NCAM immunoreactive neuronal precursor and an A2B5 immunoreactive glial precursor (Rao, M.S. and Mayer-Proschel, M. Dev. Biol. 1997 188:48-63; Rao et al. Proc. Natl Acad. Sci. USA 1998 95:3996-4001).

To determine if similar neuronal precursors could be isolated from mouse neural tubes, cells from mouse neural tubes at embryonic day 12.0 (E12.0) were analyzed for the presence of E-NCAM immunoreactive cells. A significant proportion of E12.0 neural tubes cells were E-NCAM immunoreactive (ranging from 70-80% in three independent experiments). Immunoselected E-NCAM⁺ cells divided in culture and expressed neuronal, but not glial, markers. Thus, based on their antigenic profile, E-NCAM immunoreactive cells appeared to be dividing neuron-restricted precursor cells.

To test the differentiation potential of E-NCAM immunoreactive cells, purified populations of E-NCAM⁺ cells were analyzed in mass and clonal culture. E-NCAM⁺ cells, when allowed to differentiate by addition of retinoic acid and reduction of FGF concentration, readily differentiated into multiple kinds of neurons as assessed by RT-PCR and immunocytochemistry. Further, E-NCAM⁺ cells failed to differentiate into either oligodendrocytes or astrocytes when grown under glial promoting conditions, indicating that these cells are restricted or limited in their differentiation potential.

Fura-2 Ca²⁺ imaging techniques were used to examine the ability of differentiated E-NCAM⁺ cells to respond to neurotransmitters and elevated K⁺. E12.0 E-NCAM⁺ cells were grown in culture for 10 days and allowed to differentiate. They were then loaded with fura-2 and changes in internal Ca²⁺ concentrations in response to stimulus application were monitored. Most cells (>95%) responded to external application of glutamate or acetylcholine. Smaller fractions of cells responded to dopamine, GABA or glycine. A substantial fraction of the cells responded to more than one neurotransmitter, demonstrating heterogeneity in the neuronal population derived from E-NCAM⁺ precursors. Further traces of internal Ca²⁺ changes were plotted over time for two different cells from the same clonally derived culture. While both cells responded to glutamate, acetylcholine and high K⁺, one of the cells also responded to dopamine, indicating that the receptor expression profile of cells in the same culture varies. Thus, E-NCAM immunoreactive cells isolated from E12.0 mouse neural tube are neuron-restricted precursor cells that can differentiate into a heterogeneous population of neurons, and closely resemble their rat counterparts in antigenic characteristics, differentiation properties and growth potential.

Lineage-restricted glial precursors were also isolated from the developing mouse spinal cord at E12.0 via A2B5 immunoreactivity. A2B5 immunoreactive cells were isolated by immunopanning and mass cultures of cells were tested for cell division by BrdU incorporation. A significant proportion of these cells divided in culture, thus indicating that these are a mitotic population. The antigenic properties and differentiation potential of these cells was also examined. In contrast to rat glial restricted precursor cells, it was found that a small subset of the mouse A2B5 immunoreactive cells (<5%) were β-111 tubulin immunoreactive. This population was also E-NCAM immunoreactive as determined by immunopanning experiments. Thus, unlike rat spinal cord cultures, the A2B5 epitope was not uniquely present on glial cells.

To determine if the E-NCAM/β-111 tubulin negative subset of A2B5 cells represented glial precursors, this population was examined by sequential panning. E-NCAM⁺ immunoreactive cells were isolated and discarded and the E-NCAM⁻ population was repanned to isolate A2B5 immunoreactive cells. Pooled cells were analyzed by PCR and immunocytochemistry. A2B5⁺/E-NCAM⁻ cells differentiated into oligodendrocytes and two kinds of astrocytes but failed to differentiate into neurons when grown under neuron promoting conditions thus indicating that these cells are limited in their differentiation potential. Accordingly, glial-restricted and neuron-restricted precursors are both present in the developing mouse spinal cord and these precursor cells can be distinguished by E-NCAM and A2B5 immunoreactivity.

Mouse ES cells have been shown to be capable of differentiating into neurons, astrocytes, and oligodendrocytes. In addition, it has recently been demonstrated that NEP-like cells can be harvested from ES cells, thereby bypassing the need to harvest NEP cells from fetal tissue. Accordingly, mouse ES cells cultures were analyzed to determine if more restricted neural precursors that appear at developmentally later embryonic stages could be harvested in similar fashion.

In these experiments, the ES-D3 cell line, which has been shown to be able to contribute to all cell lineages, was obtained from ATCC and grown in non-differentiating (as aggregates in DMEM/F12, 10% FCS and LIF 10 ng/ml and as adherent cultures on fibronectin coated dishes in NEP basal medium) and differentiating conditions (as adherent cultures on poly-L-Lysine/laminin substrate in NEP basal medium). Undifferentiated ES cells did not express E-NCAM immunoreactivity, but upon aggregation and treatment with retinoic acid (RA), a subset (approx. 5%) of cells began to express E-NCAM immunoreactivity. E-NCAM⁺ cells were a mitotic population as assessed by BrdU incorporation. E-NCAM⁺ cells co-expressed MAP2, a marker for neurons (DeCamilli et al. Neuroscience 1984 11:817-846) and β-111 tubulin immunoreactivity, but did not express GFAP, GalC or 04 immunoreactivity. A subset of the E-NCAM cells were nestin immunoreactive, but none of the cells expressed GFAP, a glial marker. The co-expression of neuronal markers with E-NCAM and the absence of glial markers indicate that ES cell derived E-NCAM⁺ immunoreactive cells are similar to E12.0 mouse neuronal restricted precursor cells.

To further confirm the neuronal differentiation of ES cell derived E-NCAM cells, ES cells were induced to differentiate and E-NCAM immunoreactive cells were selected by immunopanning. Cells were grown in mass or clonal culture in medium supplemented with FGF and NT-3. When mass cultures of E-NCAM cells were induced to differentiate by withdrawal of FGF and the addition of RA, cells became postmitotic, elaborated extensive processes and synthesized multiple neurotransmitters. Immunocytochemistry and PCR analysis showed the presence of various excitatory, inhibitory and cholinergic neurotransmitters after differentiation. E-NCAM immunoreactive cells failed to differentiate into either oligodendrocytes or astrocytes when grown under glial promoting conditions, indicating that these cells are limited in their differentiation potential. Thus ES cells can be used as a source of neuron-restricted precursor cells.

The differentiation potential of A2B5⁺/E-NCAM⁻ cells was tested by inducing ES cells to differentiate and depleting E-NCAM immunoreactive cells by immunopanning. E-NCAM immunonegative cells were reselected for A2B5 immunoreactivity. This double selection procedure was utilized since, as described above, A2B5⁺ immunoreactivity has been detected on glial precursors, as well as on subsets of neurons, from mice. A2B5 immunopositive cells were grown in mass or clonal culture in medium supplemented with FGF and PDGF. When mass cultures of A2B5⁺ cells were induced to differentiate by withdrawal of FGF, cells differentiated into different glial populations. Two kinds of astrocytes could be identified as early as seven days after induction of differentiation. An A2B5⁺/GFAP⁻ flat astrocyte which appeared similar to astrocytes previously characterized as Type I astrocytes and a A2B5⁺/GFAP⁺ astrocyte which appeared similar to type 2 astrocytes were detected in cultures. In addition, when cells were allowed to differentiate for longer time periods (10 days), oligodendrocyte differentiation was detected as assessed by GalC immunocytochemistry. A2B5 immunoreactive cells failed to differentiate into neurons when grown under neuron promoting conditions, indicating that these cells are limited in their differentiation potential to glial lineages. The ability of ES cell derived A2B5 immunoreactive cells to differentiate into astrocytes and oligodendrocytes confirms their resemblance to E12.0 A2B5 derived GRP cells, and indicates that like neuron-restricted precursors, glial restricted precursors can be isolated directly from differentiating ES cell cultures.

Accordingly, provided are pure populations of mouse neuroepithelial (NEP) cells isolated or derived from mouse neural tubes at embryonic day 8.5 and mouse neuron- and glial-restricted precursor cells isolated or derived from mouse neural tubes at embryonic day 12.0 or mouse ES cells which exhibit similar characteristics to the rat NEP cells described by Kalyani et al. (Dev. Biol. 1997 186:202-223) and rat GRP and rat NRP cells identified by Mayer-Proschel et al. (Neuron 1997 19:773-785) and Kalyani et al. (J. Neurosci. 1998 18:7856-7868) and Rao, M. S. and Mayer-Proschel, M. (Dev. Biol. 1997 188:48-63), Mayer-Proschel et al. (Neuron 1997 19:773-785) and Rao et al. (Proc. Natl. Acad. Sci. USA 1998 95:3996-4001), respectively. For purposes of the present invention, by "pure" it is meant a population of cells in which greater than 95%, more preferably 99%, exhibit the same characteristics.

For example, as demonstrated herein, the mouse NEP cells of the present invention grow in adherent culture, do not exhibit EGF-R immunoreactivity, do not survive or proliferate in EGF and absolutely require FGF for both proliferation and survival. PDGF, an instructive molecule for cortical stem cells, had no observable effect either alone or in combination with FGF. Under differentiating conditions, the mouse NEP cells differentiate into CNS neuronal cells, CNS glial cells including oligodendrocytes, A2B5-positive astrocytes, and A2B5-negative astrocytes, as well as neural crest stem cells.

Like rat NRP cells, the mouse NRP cells are E-NCAM immunoreactive. Further, E-NCAM appears to be expressed exclusively by the NRP cells at this stage in development as neither NEP cells nor glial precursors express E-NCAM epitopes at E12.0 stage of development. Both mouse and rat cells also proliferate in response to FGF-1 and 2 and can differentiate into multiple classes of neurons. Some small differences in proliferation and differentiation potential were noted as rat NRP cells appear more robust and survive over many more passages as compared to the more fragile mouse cell which could only be maintained for 10-12 passages. Further, rat NRP cells appear to be more heterogeneous as assessed by Ca²⁺ imaging (Kalyani et al. J. Neurosci. 1998 18:), while mouse cells show less heterogeneity and appear to mature more slowly.

Mouse GRP cells of the present invention, like rat GRP cells, generate oligodendrocytes and two kinds of astrocytes (A2B5⁺/GFAP⁺ and A2B5⁺/GFAP⁻). Like rat GRPs and unlike O-2A cells, mouse GRPs respond to CNTF by differentiating into astrocytes rather than oligodendrocytes.

Also provided are methods for isolating pure populations of these mouse NEP from mouse neural tubes at embryonic day 8.5, as well as methods for isolating pure populations of the mouse NRP and GRP cells from mouse neural tubes at embryonic day 12.0 or from differentiating mouse embryonic stem cells.

To isolate pure populations of mouse NEP cells, the neural tube from a mouse embryo at embryonic day 8.5 is removed. To isolate pure populations of mouse NRP or GRP cells, the neural tube from a mouse embryo at embryonic day 12.0 is removed. In both methods, cells from the neural tube are then dissociated in accordance with well known procedures such as is described in Example 2. The dissociated cells are then plated onto fibronectin coated cell culture plates in a medium containing fibroblast growth factor and chick embryo extract. The plated cells are then cultured at a temperature and in an atmosphere known to be conducive to growth of most cells.

Cells grown from the mouse neural tube removed at embryonic day 8.5 comprise a pure population of mouse NEP cells.

To isolate pure populations of mouse NRP cells, E-NCAM immunoreactive cells are selected from the cultured, plated cells obtained from the mouse neural tube removed at embryonic day 12.0 in accordance with well known E-NCAM immunoselection procedures such as described in Example 4. To isolate pure populations of mouse GRP cells, cells which are not E-NCAM immunoreactive, but which are A2B5-immunoreactive cells are selected from the cultured, plated cells obtained from the mouse neural tube removed at embryonic day 12.0 in accordance with well known A2B5 immunoselection procedures such as described in Example 4.

The E-NCAM and A2B5 immunoselection procedures can also be used to isolated pure populations of mouse NRP and GRP cells from mouse ES cells which have been induced to differentiated. In these methods, mouse ES cells are first plated onto dishes and exposed to conditions which induce differentiation. Various conditions which induce differentiation of ES cells have been described. In one embodiment, the ES cells are plated onto poly L-Lysine/laminin double coated dishes as described in Example 3. The differentiating cells are then subjected to E-NCAM immunoselection to isolate NRP cells or A2B5 immunoselection to isolate GRP cells. Use of mouse ES cells to isolate pure populations of mouse NRP and GRP cells of the present invention provides for a culture model for controlled, *in vitro* differentiation from a totipotent stem cell that can be readily grown and passaged over multiple generations. The mouse ES cell is amenable to perturbation, allowing the process of lineage restriction and differentiation to be characterized in detail.

The ability to isolate and grow purified populations of these mouse cells has multiple utilities. For example, these cells can be used in mouse models to develop new transplantation techniques, as well as therapeutically in humans for transplantation in diseases characterized by neuronal and/or glial degeneration. These cells are also useful in identifying new drugs which enhance survival and proliferation of these cells upon transplantation.

The cells of the present invention can also be used in the identification of genes specific to selected stages of development. In one embodiment, the cells can serve as a source of mRNA for generation of cDNA libraries that are specific to the stage development of the cells.

The cells can also be used in the generation of cell-specific antibodies for use therapeutically and diagnostically.

The following nonlimiting examples are provided to further illustrate the present invention.

### EXAMPLES

### Example 1: Substrate preparation

Fibronectin (Sigma) was diluted to a concentration of 20 *µ*g/ml in distilled H₂O (Sigma). Fibronectin solution was applied to tissue culture dishes for a minimum of 4 hours. Laminin (Biomedical Technologies Inc.), used at a concentration of 20 *µ*g/ml, was dissolved in DPBS (Gibco-BRL). To prepare fibronectin-laminin double coated dishes, laminin was applied to fibronectin coated dishes and plates were incubated overnight at 4°. Excess laminin was withdrawn and the plates were rinsed with medium.

### Example 2: Mouse cell cultures

Mouse C57BI6 embryos were removed at embryonic day 8.5 and placed in a petridish containing DPBS (Gibco-BRL). The trunk segments of embryos were enzymatically treated with collagenase (Worthington; 10 mg/ml) and dispase (Boehringer Mannheim; 20 mg/ml) for 10 minutes at room temperature. The enzyme solution was replaced with fresh medium with 10% chick embryo extract. The trunk segments were gently triturated to release the spinal cords from surrounding tissue. The spinal cords were then incubated in 0.05% trypsin solution for 5 minutes at 37°C. The cells were dissociated and plated in 35 mm fibronectin coated dishes as mass cultures (5000 cells/dish). Cells were maintained at 37°C in 5% CO₂/95% air in NEP basal medium and 10% CEE (Chicken Embryo Extract).

The NEP basal medium used in all experiments described herein is a chemically defined medium modified from that described by Stemple, D.L. and Anderson, D.J. (Cell 1992 71:973-985). The medium consists of DMEM-F12 (Gibco-BRL) supplemented with additives described by Bottenstein, J.E. and Sato, G. (Proc. Natl. Acad. Sci. USA. 1979 76:514-517) and bFGF (25 ng/ml). The NEP complete medium is NEP basal medium with 10% CEE (Chicken Embryo Extract).

Mouse C57B6 embryos were removed at embryonic day 12.0 and placed in a petridish containing DPBS. Spinal cords were mechanically dissected from surrounding connective tissue using sharpened No. 5 forceps. Isolated spinal cords were incubated in 0.05% trypsin solution for 30 minutes. The trypsin solution was replaced with fresh medium. The spinal cords were gently triturated with a pasteur pipette to dissociate the cells. The dissociated cells were plated on fibronectin/laminin coated dishes. Cells were maintained at 37° in 5% CO₂/95% air in NEP complete medium.

Differentiation into neural crest was promoted by adding BMP-4 (10 ng/ml) to the mouse NEP cells growing on fibronectin for a period of 4 days. The cells were then replated on fibronectin/laminin coated dishes in neural crest medium [NEP basal medium supplemented with bFGF (10 ng/ml), NGF (50 ng/ml), EGF (100 ng/ml)]. To promote maturation into Schwaan cells, dibutryl cAMP (5 *µ*m/ml, Sigma) was added for an additional 7 days. To promote smooth muscle differentiation, crest cells were grown in neural crest medium supplemented with 10% fetal bovine serum (Hyclone, Utah).

### Example 3: ES cell cultures

Undifferentiated ES-D3 cells (ATCC) were grown as aggregates in suspension dishes (Nunc) in DMEM-F12 with 10% FCS and LEF (Leukemia Inhibitory Factor, 10 ng/ml) for 4 days. The medium was then changed to NEP basal medium and the cells were plated on fibronectin. The medium was changed every 2 days. Differentiation into NRPs and GRPs was induced by plating the cells on poly L-Lysine/laminin double coated dishes. For long term neuronal differentiation FGF was withdrawn and Retinoic acid (Sigma) was added to the medium. Similarly for long term glial induction, cells were maintained in PDGF (10 ng/ml) and T3 (30 nm) with the withdrawal of FGF.

### Example 4: Immunopanning of E-NCAM⁺ and A2B5⁺ cells

E-NCAM⁺ and A2B5⁺ cells were purified from dissociated E 12.0 neural tube cells using a specific antibody capture assay (Wysocki, L. J., and Sato,V. L. Proc. Natl.Acad. Sci. USA 1978 75:2844-2848) with minor modifications. Specifically, the cells were trypsinized and 10 ml of cell suspension (Basal medium and 20% FCS) was plated on a 100 mm petridish and incubated at 37°C in a 5% CO₂ humidified atmosphere for 2-3 hours for the maximum attachment of flat cells. The supernatant, containing an enriched population of neuronal and oligodendrocyte cells, was plated on dishes coated with either E-NCAM antibody (5A5, Developmental Studies Hybridoma Bank (DSHB), 1: 1 dilution) or A2B5 antibody (ATCC, 1:1 dilution) to allow binding of all E-NCAM⁺ and A2B5⁺ cells. E-NCAM and A2B5 dishes were prepared by sequentially coating petridishes with an unlabeled antimouse IgM antibody (10 µg/ml) overnight, rinsing 3x with DPBS, followed by incubation with E-NCAM or A2B5 hybridoma, supernatants for 1-3 hours at room temperature. Cells were allowed to bind to the dish for 1 hour at room temperature. The supernatant was discarded and the dish was washed 8x with DPBS. The bound cells were mechanically scraped off and plated on fibronectin/laminin coated dishes in 1 ml of NEP basal medium either as mass (5000 cells/dish) or clonal (100 cells/dish) cultures. Growth factors were added every other day. In all cases, an aliquot of cells was tested for panning efficiency by immunocytochemistry. In general, the panning efficiency was greater than 90%. For E-NCAM panned OR NRP cultures, the NEP basal medium was supplemented with the following growth factors; NT3, PDGF, BDNF, and FGF (all at 10 ng/ml). For A2B5 panned or GRP cultures, the NEP basal medium was supplemented with PDGF (10 ng/ml), and FGF (10 ng/ml).

### Example 5: Immunocytochemistry

Staining procedures were in accordance with procedures described by Rao, M. S. and Mayer-Proschel, M. (Dev. Biol. 1997 188:48-63). Staining for the cell surface markers p75, E-NCAM, and GalC was carried out in cultures of living cells. To stain cells with antibodies against cytoplasmic antigens, cultures were fixed with 2% formaldehyde for 20 minutes at room temperature.

In general, dishes were incubated with primary antibody for one hour followed by incubation with an appropriate secondary antibody for 30 minutes. Double labeling experiments were performed by simultaneously incubating cells in appropriate combinations of primary antibodies followed by non-crossreactive secondary antibodies. DAPI histochemistry was performed as described by Kalyani et al.(Dev. Biol. 1997 186:202-223). DAPI (Sigma) staining was generally done after labeling had been completed. p75, E-NCAM, A2B5, and GalC were hybridoma supernatants obtained from DSHB. β-111 tubulin (Sigma), which stains neurons, nestin (monoclonal obtained from DSHB; polyclonal obtained from Signal Pharmaceuticals), a marker for undifferentiated stem cells (Zimmerman et al. Neuron 1994 12(6): following 1388; Lendahl et al. Cell 1990 60:585-595; and Dahlstrand et al. Dev. Brain Res. 1995 84:109-129), MAP2 (Sigma), another neuronal marker were used as described previously. Antibodies to glutamate and glycine were obtained from Signature Immunologicals and used as per manufacturer's recommendations. Antibodies to GFAP and GAD were obtained from Chemicon and used at 1:500 dilution.

### Example 6: BrdU incorporation

To assess the proliferation of neuronal and oligodendrocyte precursor cells, 5 bromo-2'deoxyuridine (BrdU, Sigma) at a concentration of 10 µm was added to the cells for 24 hours. The cells were then fixed with 2% paraformaldehyde for 15 minutes at room temperature followed by 95% ethanol for 30 minutes at -20°C. Cells were then washed 3x with PBS and 5% goat serum, and permeabilized with 2N HCl for 10 minutes. Acid was removed by 3 washes with PBS and 5% goat serum and the residual HCl was neutralized with 0.1 M sodium borate for 10 minutes. After rinsing with PBS cells were incubated with anti-BrdU antibody (1: 100, Sigma) for 30 minutes at room temperature in buffer containing 0.5% TRITON X-100. The cells were then incubated with goat anti-mouse IgG (1:100, Jackson Immunologicals) for 30 minutes. After 3 washes with PBS, the cells were observed with a Zeiss Fluorescence microscope.

### Example 7: RNA extraction and cDNA synthesis

Total RNA was isolated from cells or neural tubes by a modification of the guanidinium-isothiocyanate extraction method (TRIzol, Gibco-BRl). cDNA was synthesized using 1-5 µg of total RNA in a 20 µl reaction. Superscript II (Gibco-BRL), a modified Maloney murine leukemia virus reverse transcriptase and oligo (dT) 12-18 primers were used, and the Gibco-BRL protocol was followed.

### Example 8: Polymerase Chain Reaction

Aliquots of cDNA, equivalent to 1/20 of the above reaction, were used in a 50 µl reaction volume. PCR amplification was performed using Elongase polymerase (Gibco-BRL). Primer sequences and cycling temperatures used for PCR amplification of receptors are shown in the following Table. The reactions were run for 35 cycles and a 10 minute incubation at 72°C was added at the end to ensure complete extension.

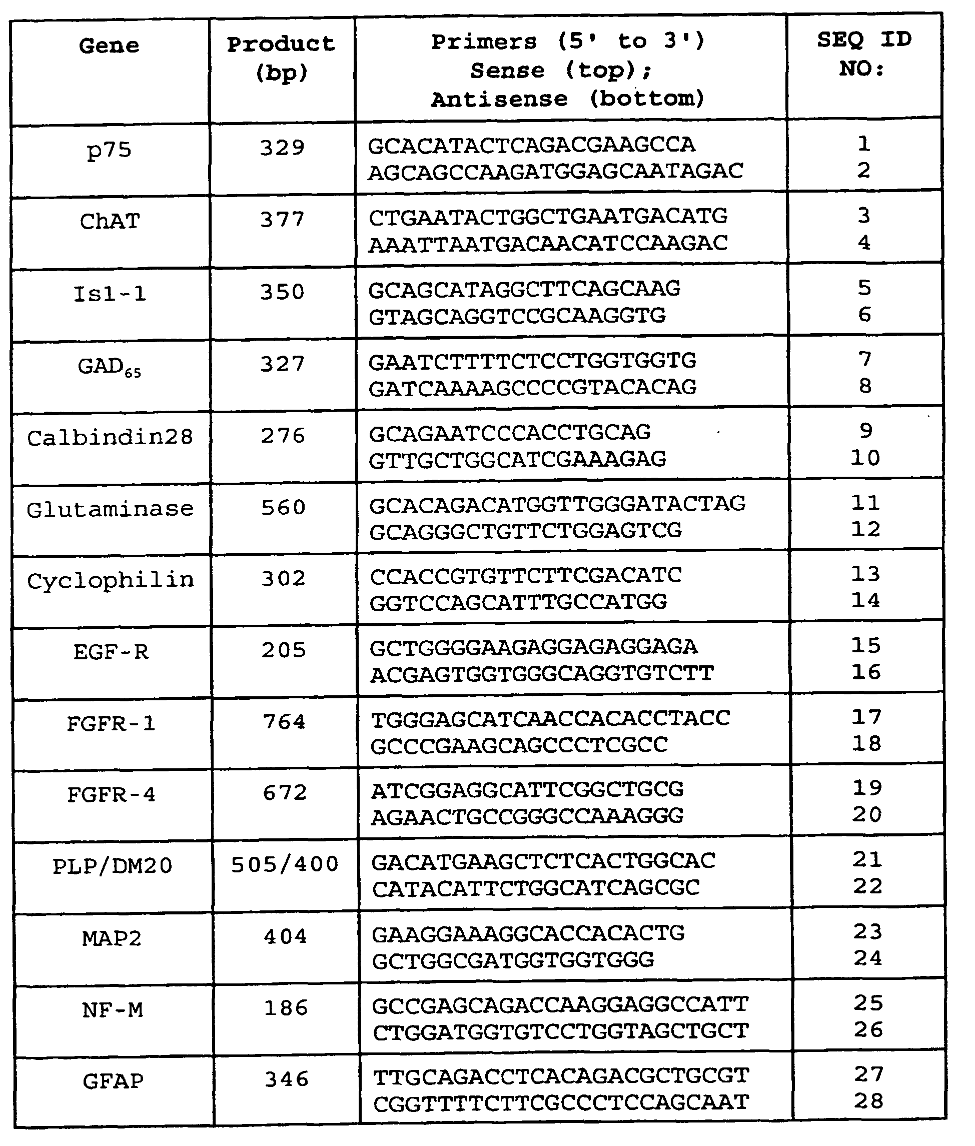

### Example 9: Intracellular Ca²⁺ Measurement

Calcium imaging experiments were performed on clonal cultures of E-NCAM panned cells obtained from mouse E8.5 embryos as described above. Cells were loaded with 5 *µ*m Fura-2 AM (Sigma, Grynkiewics *et al.*, 1985) plus pluronic F127 (Sigma, 80 µg/ml) in rat Ringer's solution (RR) at 23°C in the dark. Following the 20 minute incubation, cells were washed three times in RR and allowed to de-esterify for 30 minutes. Relative changes in intracellular Ca²⁺ concentrations were measured from the background-corrected ratio of fluorescence intensity by excitation at 340/380 nm. A response was defined as a minimum rise of 10% of the ratioed baseline value. A Zeiss-Attofluor imaging system and software (Atto Instruments Inc.) were used to acquire and analyze the data. Data points were sampled at 1 Hz. Neurotransmitters (100 µM concentration) were made fresh in RR and delivered by bath exchange using a small volume loop injector (200 *µ*l). RR consisted of (in mM): 140 NaCl, 3 KCl, MgCl₂, 2 CaCl₂, 10 HEPES, and 10 glucose. Ascorbic acid (500 *µ*M) was added to dopamine solutions to prevent oxidation. Control application of 500 *µ*M ascorbic acid had no effect. The pH of all solutions was adjusted to 7.4 with NaOH. High K⁺ RR was made by substituting 50 mM K⁺ for Na⁺ in the normal RR.

## Claims

1. An isolated, pure population of mouse glial-restricted precursor cells derived from mouse neural tubes at embryonic day 12.0 or mouse embryonic stem cells by A2B5⁺ immunoreactivity.

2. A method for isolating a pure population of the mouse glial-restricted precursor cells of claim 1 comprising:
(a) incubating mouse embryonic stem cells under differentiation-inducing conditions so that the cells differentiate; and
(b) isolating a pure population of mouse glial-restricted precursor cells by immunoselecting A2B5-immunoreactive cells from the differentiated cells.

3. A method for isolating a pure population of the mouse glial-restricted precursor cells of claim 1 comprising:
(a) removing a neural tube from a mouse embryo at embryonic day 12.0;
(b) dissociating cells from the neural tube;
(c) plating the dissociated cells in a feeder-cell-independent culture on a substratum and in a medium containing fibroblast growth factor and chick embryo extract;
(d) culturing the plated cells at a temperature and in an atmosphere conducive to cell growth; and
(e) isolating a pure population of mouse glial-restricted precursor cells by immunoselecting A2B5-immunoreactive cells from the cultured, plated cells.

## Revendications

1. Population pure, isolée, de cellules précurseurs à restriction gliale de souris, dérivées de tubes neuraux de souris au jour embryonnaire 12.0 ou de cellules souches embryonnaires de souris par immunoréactivité A2B5⁺.

2. Procédé pour isoler une population pure des cellules précurseurs à restriction gliale de souris de la revendication 1, comprenant les opérations consistant à :
(a) faire incuber des cellules souches embryonnaires de souris dans des conditions induisant une différenciation de telle sorte que les cellules se différencient ; et
(b) isoler une population pure de cellules précurseurs à restriction gliale de souris par immunosélection de cellules à immunoréactivité A2B5 à partir des cellules différenciées.

3. Procédé pour isoler une population pure des cellules précurseurs à restriction gliale de souris de la revendication 1, comprenant les opérations consistant à :
(a) retirer un tube neural provenant d'un embryon de souris au jour embryonnaire 12.0 ;
(b) dissocier des cellules provenant du tube neural ;
(c) étaler en boîte de Pétri les cellules dissociées dans une culture indépendante des cellules nourricières sur un substratum et dans un milieu contenant le facteur de croissance des fibroblastes et un extrait d'embryon de poulet ;
(d) cultiver les cellules étalées en boîte de Pétri à une température et dans une atmosphère contribuant à une croissance cellulaire ; et
(e) isoler une population pure de cellules précurseurs à restriction gliale de souris par immunosélection de cellules à immunoréactivité A2B5 provenant des cellules étalées en boîte de Pétri, cultivées.

## Patentansprüche

1. Isolierte, reine Population von Glia-begrenzten Vorläuferzellen der Maus, die aus Neuralrohren der Maus am Embryonaltag 12,0 oder aus embryonalen Stammzellen der Maus durch A2B5⁺-Immunreaktivität abstammen.

2. Verfahren zur Isolierung einer reinen Population der Glia-begrenzten Vorläuferzellen der Maus aus Anspruch 1, umfassend:
(a) Inkubieren von embryonalen Stammzellen der Maus unter Bedingungen, die Differenzierung induzieren, so dass sich die Zellen differenzieren; und
(b) Isolieren einer reinen Population von Glia-begrenzten Vorläuferzellen der Maus durch Immunselektieren von A2B5-immunreaktiven Zellen aus den differenzierten Zellen.

3. Verfahren zur Isolierung einer reinen Population der Glia-begrenzten Vorläuferzellen der Maus aus Anspruch 1, umfassend:
(a) Entnahme eines Neuralrohrs aus einem Mäuseembryo am Embryonaltag 12,0;
(b) Abtrennen der Zellen vom Neuralrohr;
(c) Anlegen einer Plattenkultur der abgetrennten Zellen in einer Ernährungszellen-unabhängigen Kultur auf einem Nährboden und in einem Medium, das Fibroblastenwachstumsfaktor und Hühnerembryoextrakt enthält;
(d) Züchten der auf Platten kultivierten Zellen bei einer Temperatur und in einer Atmosphäre, die das Zellwachstum fördern; und
(e) Isolieren einer reinen Population von Glia-begrenzten Vorläuferzellen der Maus durch Immunselektieren von A2B5-immunreaktiven Zellen aus den gezüchteten, auf Platten kultivierten Zellen.
